# EUROPÄISCHE PATENTSCHRIFT

(11) **EP 3 777 904 B1**
(45) Veröffentlichungstag und Bekanntmachung des Hinweises auf die Patenterteilung: **13.04.2022**
(21) Anmeldenummer: 19192028.9
(22) Anmeldetag: 16.08.2019
(51) Int. Cl.: A61L 27/12, C01B 25/32

(54) **OSTEOTROPER KNOCHENERSATZ**
OSTEOTROPIC BONE REPLACEMENT
SUBSTITUTION OSSEUSE OSTÉOTROPE

(43) Veröffentlichungstag der Anmeldung: 17.02.2021
(73) Patentinhaber: Universität Heidelberg, 69117 Heidelberg (DE); Haas, Andreas, 69207 Sandhausen (DE)
(72) Erfinder: Haas, Andreas, 69207 Sandhausen (DE); Kasperk, Christian, 69123 Heidelberg (DE); Burchard, Michael, 69412 Eberbach (DE)
(74) Vertreter: Wunderlich & Heim Patentanwälte Partnerschaftsgesellschaft mbB

(56) Entgegenhaltungen:
- EP-A1- 2 228 080
- CN-A- 101 928 136
- CN-A- 105 712 737
- TW-A- 201 200 172
- TW-A- 201 200 471
- P. MELNIKOV ET AL: "Gallium-containing hydroxyapatite for potential use in orthopedics", MATERIALS CHEMISTRY AND PHYSICS, Bd. 117, Nr. 1, 1. September 2009 (2009-09-01), Seiten 86-90, XP055497554, Switzerland, Taiwan, Republic of China ISSN: 0254-0584, DOI: 10.1016/j.matchemphys.2009.05.046
- DATABASE WPI Week 201127 Thomson Scientific, London, GB; AN 2011-B19964 XP002797327, & CN 101 928 136 A (CUI S) 29. Dezember 2010 (2010-12-29)
- SAKAI AKIKO ET AL: "Preparation of Sr-containing carbonate apatite as a bone substitute and its properties", DENTAL MATERIALS JOURNAL, vol. 31, no. 2, 1 January 2012 (2012-01-01), pages 197-205, XP55793154, JP ISSN: 0287-4547, DOI: 10.4012/dmj.2011-177
- E. LANDI ET AL: "Sr-substituted hydroxyapatites for osteoporotic bone replacement", ACTA BIOMATERIALIA, ELSEVIER, AMSTERDAM, NL, vol. 3, no. 6, 14 September 2007 (2007-09-14), pages 961-969, XP022245491, ISSN: 1742-7061, DOI: 10.1016/J.ACTBIO.2007.05.006
- BOANINI E ET AL: "Osteopenic bone cell response to strontium-substituted hydroxyapatite", JOURNAL OF MATERIALS SCIENCE: MATERIALS IN MEDICINE, KLUWER ACADEMIC PUBLISHERS, BO, vol. 22, no. 9, 21 June 2011 (2011-06-21), pages 2079-2088, XP019947831, ISSN: 1573-4838, DOI: 10.1007/S10856-011-4379-3
- MELLIER CHARLOTTE ET AL: "Design and properties of novel gallium-doped injectable apatitic cements", ACTA BIOMATERIALIA, ELSEVIER, AMSTERDAM , NL, vol. 24, 12 June 2015 (2015-06-12), pages 322-332, XP029868093, ISSN: 1742-7061, DOI: 10.1016/J.ACTBIO.2015.05.027
- MELNIKOV P ET AL: "Gallium-containing hydroxyapatite for potential use in orthopedics", MATERIALS CHEMISTRY AND PHYSICS, ELSEVIER SA, SWITZERLAND, TAIWAN, REPUBLIC OF CHINA, vol. 117, no. 1, 15 September 2009 (2009-09-15), pages 86-90, XP026305348, ISSN: 0254-0584, DOI: 10.1016/J.MATCHEMPHYS.2009.05.046 [retrieved on 2009-06-16]
- QU ET AL: "The effect of fluoride contents in fluoridated hydroxyapatite on osteoblast behavior", ACTA BIOMATERIALIA, ELSEVIER, AMSTERDAM , NL, vol. 2, no. 1, 1 January 2006 (2006-01-01) , pages 113-119, XP005221584, ISSN: 1742-7061, DOI: 10.1016/J.ACTBIO.2005.09.003
- Jennifer H. Shepherd ET AL: "Substituted hydroxyapatites for bone repair", Journal of Materials Science: Materials in Medicine, 1 January 2012 (2012-01-01), XP055034846, ISSN: 0957-4530, DOI: 10.1007/s10856-012-4598-2
- ÖZBEK YILDIZ YARALI ET AL: "Synthesis and characterization of strontium-doped hydroxyapatite for biomedical applications", JOURNAL OF THERMAL ANALYSIS AND CALORIMETRY, KLUWER, DORDRECHT, NL, vol. 125, no. 2, 16 June 2016 (2016-06-16) , pages 745-750, XP035996075, ISSN: 1388-6150, DOI: 10.1007/S10973-016-5607-3 [retrieved on 2016-06-16]
- LI ET AL: "Chemical composition, crystal size and lattice structural changes after incorporation of strontium into biomimetic apatite", BIOMATERIALS, ELSEVIER, AMSTERDAM, NL, vol. 28, no. 7, 22 December 2006 (2006-12-22), pages 1452-1460, XP005812902, ISSN: 0142-9612, DOI: 10.1016/J.BIOMATERIALS.2006.11.001

## Beschreibung

Die Erfindung betrifft ein Verfahren zum Herstellen eines osteotropen Knochenersatzmaterials aus einem Ausgangsmaterial, welches im Wesentlichen Portlandit, Ätzkalk, Aragonit, Kalzit, beispielsweise Skelette kalkinkrustierender Algen, und/oder Apatit, beispielsweise als Hydroxylapatit, z.B. aus Wirbeltierknochen oder synthetisch hergestellt, aufweist. Ferner betrifft die Erfindung ein osteotropes Knochenmaterial, welches entsprechend des erfindungsgemäßen Verfahrens hergestellt wurde.

Im Rahmen der Erfindung können unter den angeführten Ausgangsmaterialien auch Materialien verstanden werden, welche einen zu den Ausgangsmaterialien analoge Struktur aufweisen, jedoch synthetisch hergestellt sind.

Viele zurzeit verwendete Knochenersatzmaterialien aus natürlichem Knochen, sowohl autolog, allogen als auch xenogen, Korallen, Algen oder auch vollsynthetisch hergestellte Hydroxylapatit-Knochenersatzmaterialien, welche als Knochenimplantat beziehungsweise als Knochenersatzmaterial im Rahmen von Knochenaugmentationen verwendet werden, haben nur osteokonduktive Eigenschaften. Dies bedeutet, dass die bisherigen Calciumphosphat oder Hydroxylapatit-Knochenersatzmaterialien zwar eine geeignete biokompatible Oberfläche zum direkten Aufwachsen von Knochengewebe auf der Implantatoberfläche bieten, jedoch stimulieren sie nicht direkt die Knochenneubildung in der direkten Knochenumgebung des Implantats. Hydroxylapatit oder Calciumphosphatmaterialien sind grundsätzlich nur osteokonduktiv. Sie erlauben also das Aufwachsen von Knochen, stimulieren selbst aber nicht die Proliferation oder Ausdifferenzierung von knochenanbauvornehmenden Zellen, wie Osteoblasten oder deren Vorläufern. Unter direkten Aufwachsen im Sinne der Erfindung kann insbesondere das Aufwachsen ohne "Zwischen-Gewebeschicht" zwischen Implantatoberfläche und Knochengewebe verstanden werden.

Um eine osteoinduktive Wirkung zu entfalten werden bisher Knochenersatzmaterialien oder auch organische Substanzen, wie Kollagene oder organische Moleküle, mit weiteren Proteinen, Peptiden oder anderen Molekülen ergänzt. Beispiele hierfür sind Wachstumsfaktoren beispielsweise diverse BMPs, IGF1/2, FGF o.ä., oder Serumprodukte. Hierbei besteht die Problematik, dass diese Substanzen häufig unklare Zeiträume im Implantat beziehungsweise am Implantatort verweilen, da sie meist schnell aus dem Implantatlager weggespült oder abgebaut werden. Dadurch liegen die osteoinduktiven Eigenschaften zum einen nur kurzzeitig und nicht wirklich dosierbar vor, zum anderen sind auch systemische Wirkungen im gesamten Organismus möglich, die in der Regel nicht bezweckt sind.

Beispiele für Herstellungsverfahren für ein derartiges Knochenersatzmaterial gehen aus den europäischen Patenten EP 230 570 B1 und EP 028 074 B1 hervor. Hierbei wird ein Knochenersatzmaterial aus Hydroxylapatit, welches von kalkinstruierenden Meeresalgen stammt, gewonnen, in dem das in der Natur vorliegende Kalziumcarbonatskelett entsprechend umgewandelt wird.

Jedoch ist ein derartiges Knochenersatzmittel wie bereits beschrieben, lediglich osteokonduktiv und nicht osteoinduktiv.

Ferner gehen aus P. Melnikov et al., Materials Chemistry and Physics 117(1), 86-90, (2009) Gallium enthaltender Hydroxyapatit zur Verwendung in der Orthopädie, aus CN 101928136 A fluorierter Hydroxyapatit und seine Verwendung zur Herstellung von künstlichen Knochen und aus EP 2 228 080 A eine Gallium-dotierte Phosphocalciumverbindung mit Apatitstruktur zum Füllen von Zahn- oder Knochendefekten hervor.

Auch die XP 55 793 154 (A. Sakai et al., Dental Materials Journal 31(2), 197-205 (2012)) und die XP 0222 45 491 (E. Landi et al., Acta Biomaterialia 3, 961-969 (2007)) beschreiben einen Knochenzement aus Apatit, in dem Strontium vorhanden ist.

Der Erfindung liegt daher die **Aufgabe** zugrunde, ein Verfahren zum Herstellen eines Knochenersatzmaterials und ein Knochenersatzmaterial selbst anzugeben, welches langwirksame, örtlich begrenzte osteotrope Eigenschaften aufweist.

Diese Aufgabe wird erfindungsgemäß durch ein Verfahren zum Herstellen eines osteotropen Knochenersatzmaterials mit den Merkmalen des Anspruches 1 sowie ein osteotropes Knochenersatzmaterial mit den Merkmalen des Anspruches 13 gelöst. Vorteilhafte Ausführungsformen der Erfindung sind in den Unteransprüchen angegeben. Entsprechend der Erfindung ist vorgesehen, dass ein Ausgangsmaterial verwendet wird, welches im Wesentlichen Portlandit, Ätzkalk, Aragonit, Kalzit und/oder Apatit beispielsweise als Hydroxylapatit aufweist, wobei Portlandit, Ätzkalk, Aragonit und/ oder Kalzit in Form von gebrannten, ungebrannten und/oder chemisch aufbereiteten biogenen Skeletten verwendet werden oder wobei als Apatit Wirbeltierknochen nach pyrolytischer oder chemischer Mazeration verwendet werden.

Das Ausgangsmaterial wird mit einer Strontium-, Fluor- und/oder Galliumquelle in einen Autoklav eingebracht, wobei beim Verwenden eines Ausgangsmaterial, welches im Wesentlichen Portlandit, Ätzkalk, Aragonit; Kalzit aufweist eine Phosphatquelle eingebracht wird. Bei einem Ausgangsmaterial, welches im Wesentlichen aus Apatit besteht, kann ebenfalls eine Phosphatquelle eingebracht werden, dies ist jedoch nicht zwingend erforderlich. Zusätzlich wird Wasser als Bestandteil eines Lösungsmittels in den Autoklav zugegeben. Anschließend wird der pH-Wert bei Raumtemperatur und Normaldruck in dem Autoklav in einem Bereich über 7 eingestellt. Dann wird der geschlossene und gefüllte Autoklav für mindestens eine Stunde erhitzt und anschließend abgekühlt. Schlussendlich wird der Inhalt des Autoklavs von den Resten der Strontium-, Fluorid- und/oder Galliumquelle gereinigt. Selbiges gilt auch für die Phosphorquelle, sofern eingesetzt.

Entsprechend der Erfindung wurde erkannt, dass unter anderem Strontium, Fluorid- und/oder Galliumionen, welche in eine Apatitstruktur eingebaut werden, osteodinduktive beziehungsweise osteotrope oder antiresorptive Eigenschaften aufweisen. Wesentlich bei der Erfindung ist hierbei das erfindungsgemäße Verfahren, um die Strontium- Fluorid- und/oder Galliumionen entsprechend in Apatit, insbesondere Hydroxylapatitstruktur, einzubauen und nicht lediglich anzulagern. Bei einer rein oberflächlichen Anlagerung könnte ein Ablösen der angelagerten Substanzen auftreten. Durch das erfindungsgemäße Verfahren ist es jedoch möglich, die oben angesprochenen Ionen, die osteotrope, also osteoinduktive, antiresorptive und/oder dem Knochengewebsaufbau förderliche Eigenschaften aufweisen, derart mit der entstehenden Apatitstruktur zu verbinden, dass diese nicht ohne weiteres herausgelöst werden können.

Für eine erfolgreiche Umwandlung der Ausgangsmaterialien aus Kalziumkarbonatmaterialien. also insbesondere Portlandit, Ätzkalk, Aragonit, Kalzit, in Apatit beziehungsweise Hydroxylapatitstruktur ist das Vorhandensein einer Phosphatquelle wesentlich, wobei verschiedene Phosphatsalze eingesetzt werden können. Bei der Verwendung eines Apatitmateriales als Ausgangsmaterial ist das Vorhandensein einer Phosphatquelle nicht zwingend notwendig aber vorteilhaft. Hierdurch wird insbesondere vorgebeugt, dass die vorhandene Struktur während des Verfahrens geschwächt wird.

Besonders gute Ergebnisse konnten entsprechend den Untersuchungen, welche der Erfindung zugrunde liegen, erreicht werden, wenn der geschlossene und gefüllte Autoklav bei mindestens 30°C bevorzugt über 190°C, erhitzt wird. Dies kann in einem Ofen oder ein Heizmodul erfolgen. Es ist auch möglich ein Heizmodul integriert mit dem Autoklav vorzusehen. Bei diesen Temperaturen wird eine ausreichend gute Umwandlung des Ausgangsmaterials in dotierten-Apatit erreicht, wobei zusätzlich, wie bereits beschrieben, die genannten Ionen eingelagert und mit dem Kristallgitter des Apatits verbunden werden. Je geringer die Temperatur des Ofens, um so langsamer läuft die im Autoklav ablaufende Reaktion ab.

Als Ofen im Sinne des erfindungsgemäßen Verfahrens kann auch ein Wärmeschrank oder dergleichen bezeichnet werden. Wesentlich hierbei ist, dass das Gerät eine gewünschte Temperatur zwischen 30 und mehreren hundert Grad konstant über einen längeren Zeitraum halten kann.

Zum Reinigen des Inhalts des Autoklavs von Resten der Strontium-, Fluor-, und/oder Galliumquelle sowie der Phosphorquelle, sind verschiedene Verfahren möglich. Je nach Auswahl der Quellen kann der Inhalt des Autoklavs bevorzugt mechanisch mit einer Filtervorrichtung gereinigt werden. Dies ist der Fall, wenn möglichst große beziehungsweise schwer lösliche Quellen verwendet werden.

Für eine gute Gewebsverträglichkeit des erfindungsgemäßen Knochenersatzmaterials ist es vorteilhaft, wenn der Inhalt des Autoklavs nach einer, insbesondere mechanischen, Reinigung bis zum Erreichen eines pH-Wertes von unter 8 gereinigt wird.

Zum Einstellen des pH-Wertes vor dem Verschließen des Autoklavs in einem Bereich von über 7 kann eine basische Lösung, insbesondere eine Ammoniaklösung, verwendet werden. Beispielsweise kann hierfür eine Ammoniumdihydrogenphosphatlösung oder auch andere Phosphatverbindung verwendet werden.

Bevorzugt wird die Einbringung einer Strontium-, Fluor- und/oder Galliumquelle im Überschuss in Bezug auf das Ausgangsmaterial. Dies gilt vorteilhafterweise auch für die Phosphatquelle. Anders ausgedrückt, es wird ein großes Reservoir der entsprechenden Materialien zur Verfügung gestellt, so dass mit hoher Sicherheit ein guter Einbau in das Kristallgitter des Apatites erreicht werden kann. Im abschließenden zuvor beschriebenen Reinigungsschritt werden die entsprechenden nichtverbrauchten Ausgangsstoffe der Strontium-, Fluor- und/oder Galliumquelle sowie der Phosphorquelle aus dem hergestellten Material entfernt. Dies bedeutet, dass das Material gereinigt wird.

Bei der Verwendung von zum Beispiel Ammoniumdihydrogenphosphat oder Diammoniumphosphat als Phosphorquelle, kann dieses Mittel zugleich zum Einstellen des gewünschten pH-Wertes verwendet werden. Auch hat sich herausgestellt, dass die Verwendung von Ammoniumdihydrogenphosphat aufgelöst in Wasser besonders einfach zu dosieren ist und eine optimale Reaktionsumgebung in dem Autoklav erreicht werden kann.

Bevorzugt liegen Calcium- und Phosphor-Atome in der Gesamtmischung in der Autoklave höchstens im Verhältnis 10:5 vor. Ein derartiges Verhältnis hat sich als vorteilhaft für die osteotropen Eigenschaften des entstehenden Knochenersatzmaterials herausgestellt, da so die entsprechenden osteotropen also osteoinduktiven und/oder antiresorptiv wirksamen Stoffe in einem Verhältnis vorliegen, welches besonders gute Ergebnisse ermöglicht.

Als Apatit werden Wirbeltierknochen verwendet.

Säugetierknochen, beispielsweise von Rindern oder Schweinen, liefern bereits Apatit mit einem erheblichen Anteil von Hydroxyl- und Carbonat-Apatit. Bei dem Verwenden von Wirbeltier- oder Säugetierknochen werden diese zuvor einer pyrolytischen oder chemischen Mazeration, das heißt dem Entfernen von immunogenem Material, unterzogen.

Grundsätzlich wird ein längeres Erhitzen des geschlossenen und gefüllten Autoklavs bevorzugt. Es wurde entsprechend der Erfindung festgestellt, dass nach 1 bis 4 Tagen sehr gute Ergebnisse des entstehenden osteotropen Knochenersatzmaterials erreicht werden, so dass ein noch längeres Erhitzen nicht zwingend zu deutlich besseren Ergebnissen führt.

Wie bereits beschrieben, wird die Strontium-, Fluor- und/oder Galliumquelle nach dem Erhitzen im Autoklav und dem anschließenden Abkühlen von dem entstehenden osteotropen Knochenersatzmaterial abgetrennt, indem dieses gereinigt wird. Hierbei ist es bevorzugt, wenn als Strontium-, Fluor- und/oder Galliumquelle ein leicht auswaschbares oder schwer wasserlösliches Material verwendet wird. Die Verwendung eines leicht auswaschbaren Materials hat den Vorteil, dass dieses beim Waschen des hergestellten osteotropen Knochenersatzmaterials leicht ausgewaschen und somit das osteotrope Knochenersatzmaterial leichter gereinigt werden kann. Alternativ bietet ein schwer lösliches, insbesondere grobkristallines, Material den Vorteil, dass während des Umwandlungsprozesses im Autoklav ausreichend Strontium-, Fluor- und/oder Galliumionen zur Verfügung stehen, jedoch eine Reinigung anschließend besonders leicht, beispielsweise auch mechanisch erfolgen kann.

Ein besonders leichtes Reinigen des entstandenen osteotropen Knochenersatzmaterials kann dann erreicht werden, wenn die Strontium-, Fluor- und/oder Galliumquelle als Feststoff in einem Behältnis, in den Autoklav zugegeben werden. Hierbei ist das Behältnis derart ausgebildet, dass es den Austausch von Ionen der Strontium-, Fluor- und/oder Galliumquelle mit dem Lösungsmittel, welches im Autoklav vorhanden ist, ermöglicht, jedoch die Feststoffe zurückgehalten werden. Dies erleichtert das abschließende Reinigen des entstandenen osteotropen Knochenersatzmaterials deutlich.

Bevorzugt ist es, wenn das Ausgangsmaterial vor oder nach dem Einbringen in den Autoklav und/oder der Inhalt des Autoklavs nach dem Ablauf des Verfahrens einer pyrolytischen Behandlung und/oder einer chemischen Reinigung unterzogen wird. Die pyrolytische Behandlung beziehungsweise das chemische Reinigungsverfahren haben den Vorteil, dass eventuell vorhandene Proteine oder andere organische Fremdstoffe aus dem hergestellten osteotropen Knochenersatzmaterial entfernt werden, so dass Wechselwirkungen beim Implantieren des Knochenersatzmaterials in einen menschlichen Körper minimiert beziehungsweise ausgeschlossen werden.

Ferner betrifft die Erfindung ein osteotropes Knochenersatzmaterial, welches nach dem vorstehend beschriebenen erfindungsgemäßen Verfahren hergestellt wurde. Das osteotrope Knochenersatzmaterial, welches im Wesentlich Apatit aufweist, weist Strontium-, Fluor- und/oder Galliumionen in seinem Kristallgitter auf. Diese entfalten osteotrope Eigenschaften nach dem Implantieren in einen tierischen oder menschlichen Körper. Neben Apatit, insbesondere Hydroxylapatit, können auch geringe Bestandteile von Calziumphosphat, Calcit, Aragonit und Tricalziumphosphat vorhanden sein.

Das gemäß dem erfindungsgemäßen Verfahren hergestellte erfindungsgemäße osteotrope Knochenersatzmaterial kann beispielsweise zum Herstellen von formstabilen Blöcken verwendet werden, indem auch Zahnzylinderimplantate oder sonstige metallische Gegenstände integriert sind. Diese Blöcke können als zukünftige Zahnimplantatträger in einen Kieferknochen implantiert werden und sofort und ohne einen weiteren Zweiteingriff nach einer Heilungsphase von 3 bis 6 Monaten verwendet werden. Da das entstehende osteotrope Knochenersatzmaterial eine pulverähnliche Form aufweist, können die formstabilen Blöcke, welche aus diesem hergestellt werden, nach Maß angefertigt werden und somit auf Knochendefekte angepasst werden, wie beispielsweise eine Zahnlücke.

Durch das erfindungsgemäße Einbetten der Strontium-, Fluor- und/oder Galliumionen entfaltet das Knochenersatzmaterial ohne weitere Zugabe eine örtliche osteotrope Wirkung, welche hauptsächlich und im Wesentlichen nur im Implantatlager stattfindet. Damit hat das erfindungsgemäße Knochenersatzmaterial keine systemische Wirkung. Ein weiterer Vorteil ist, dass das erfindungsgemäße Knochenersatzmaterial seine osteoinduktiven bzw. osteotropen Wirkungen über die Gesamtzeit des Verbleibs im Implantatlager entfaltet und erst beendet, sobald es von autochtonem Knochengewebe ersetzt wird. So wird eine schnellere und nachhaltigere Knochenheilung eines Knochendefektes erreicht.

Ein anderes Anwendungsbeispiel für das erfindungsgemäße osteotrope Knochenersatzmaterial kann die Stabilisierung bei osteoporotischen, traumatischen und/oder malignombedingten Wirbelbrüchen oder Sinterungsfrakturen sein. Eine weitere Möglichkeit ist, das erfindungsgemäß hergestellte Material als Ausgangsmaterial für 3-D-Druckverfahren einzusetzen.

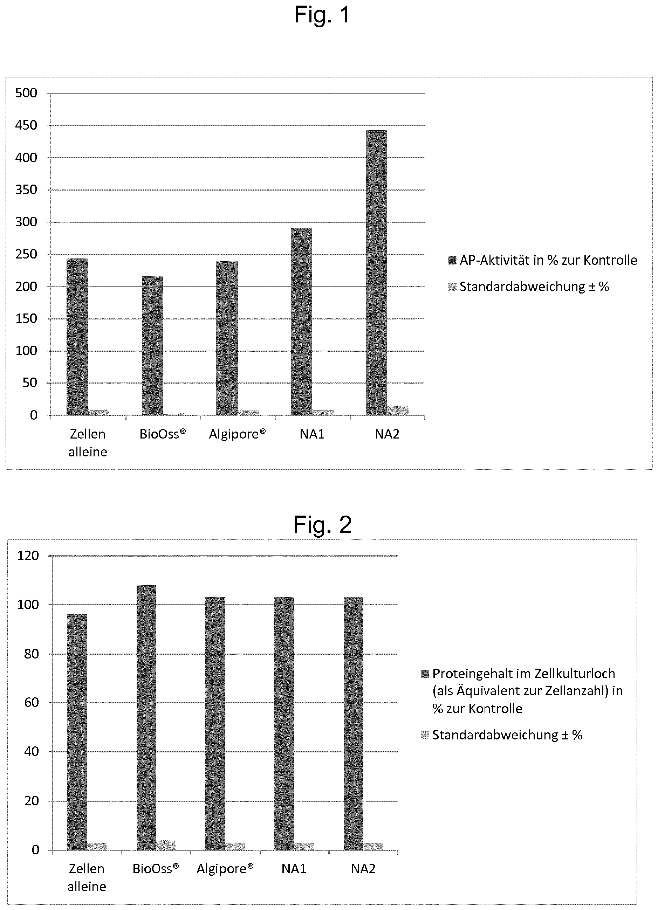

Die Erfindung wird nachfolgend anhand von schematisch exemplarischen Ausführungsbeispielen mit Bezugnahme auf die Figuren noch näher erläutert. Hierbei zeigt
- Figuren 1 bis 6: Ergebnisse der Vergleichsversuche

### Herstellungsverfahren

Im Folgenden wird eine beispielsweise Herstellung des erfindungsgemäßen osteotropen Knochenersatzmaterials entsprechend dem erfindungsgemäßen Verfahren beschrieben.

Hierfür wird ein 150 ml fassendes Teflongefäß verwendet. Dieses wird mit folgenden Substanzen gefüllt:

| | |
|---|---|
| gebranntes Algenmaterial | 19,822 g |
| Ammoniumdihydrogenphosphat | 26,38 g |
| Strontiumfluorid | 2,212 g |
| Kaliumfluorid | 2,212 g |
| Ammoniaklösung (25%ig) | 50 ml |
| deionisiertes H₂O | 50 ml |

Als Ausgangsstoff wird als ein Beispiel für Aragonit ein Skelett von kalkinkrustierenden Algen verwendet. Bei CO₂-haltigen Ausgangsmaterialien ist es meist vorteilhaft, wenn diese gebrannt werden, hierbei ist eine Erhaltung der äußeren Struktur des Materials wünschenswert. Wie beschrieben, kann das erfindungsgemäße Verfahren aber auch auf ein Apatitmaterial zum Beispiel aus Wirbeltierknochen als Ausgangsmaterial, angewendet werden, wobei sich die Anwesenheit einer Phosphatquelle als vorteilhaft einerseits für die Einbringung der osteotropen Ionen andererseits aber auch für den Erhalt der Struktur des Ausgangsmaterials erwiesen hat.

Das Algenskelett wird vor der Zugabe in das Teflongefäß ausgebrannt, damit etwaige Fremdproteine, Eiweiße oder dergleichen entfernt werden. Ferner wird Ammoniumdihydrogenphosphat, Strontiumfluorid, Kaliumfluorid und eine 25-prozentige Ammoniaklösung zugegeben. Zusätzlich wird auch deionisiertes Wasser zugefügt.

Die entsprechenden Gewichte beziehungsweise Volumina der zugeführten Stoffe können der Tabelle entnommen werden.

Hierbei dient das Ammoniumdihydrogenphosphat als Phosphatquelle, wobei wie beschrieben auch andere Phosphatquellen möglich sind. Strontiumfluorid wird als Strontiumquelle wie auch als Fluorquelle verwendet. Kaliumfluorid wird ebenfalls als Fluoridquelle eingesetzt.

Nachdem die Substanzen in das Teflongefäß eingebracht wurden und eine eventuelle Gasentwicklung abgewartet wurde, wird dieses verschlossen. Anschließend wird das Teflongefäß in einen Autoklaven beispielsweise in ein Druckaufschlussgefäß eingesetzt. Dieses Gefäß ist bevorzugt aus Edelstahl. Anschließend wird der Deckel fest verschraubt, so dass ein Autoklav entsteht.

Das entsprechend fest verschlossene Druckaufschlussgefäß wird anschließend in einen vorgeheizten Wärmeschrank beziehungsweise einen Heizblock eingesetzt, welcher eine Temperatur von 190°C aufweist.

Das Druckaufschlussgefäß verbleibt in dem Wärmeschrank für 5 Tage, wobei die Temperatur von 190°C aufrechterhalten wird. Nach Ablauf dieser Zeit wird der Wärmeschrank abgeschaltet. Das Druckaufschlussgefäß kühlt dann im Wärmeschrank beziehungsweise im Heizblock langsam ab. Dies nimmt etwa einen Tag in Anspruch.

Nach vollständigem Abkühlen wird das Druckaufschlussgefäß und das Teflongefäß geöffnet und das entstandene osteotrope Knochenersatzmaterial gereinigt. Hierfür wird das osteotrope Knochenersatzmaterial mit Wasser auf ein Filterpapier aufgebracht und gewaschen. Es werden mehrere Reinigungsvorgänge, beispielsweise Spühlvorgänge, durchgeführt, bis sich ein pH-Wert von unter 8 einstellt.

Anschließend wird das osteotrope Knochenersatzmaterial erneut in den Wärmeschrankt eingebracht, jedoch nur bei 40°C getrocknet.

Danach liegt das osteotrope Knochenersatzmaterial zur weiteren Verwendung bereit. Es kann dann beispielsweise in gewünschte Formen gebracht und sterilisiert werden.

### Versuche und Ergebnisse

Im Folgenden wird die Wirkung des neuen osteotropen Knochenersatzmaterials, welches entsprechend dem erfindungsgemäßen Verfahren hergestellt wird, näher erläutert beziehungsweise anhand von Versuchsergebnissen die osteoinduktive Wirksamkeit belegt. Die Ergebnisse zeigen, dass durch das erfindungsgemäße Knochenersatzmaterial die lokale Knochenneubildung im Knochendefekt zusätzlich stimuliert werden sollte, da bei menschlichen Knochenzellen der wichtigste Knochenanbaumarker, die alkalische Phosphatase, stimuliert wird.

Es wurden in vitro Versuche mit primären menschlichen osteoblastären Knochenzellen durchgeführt, um den direkten Einfluss des erfindungsgemäßen osteotropen Knochenersatzmaterials im Kontakt zu primären menschlichen Zellen zu untersuchen. Dies wurde auch durchgeführt, um schädliche Einflüsse des erfindungsgemäßen osteotropen Knochenersatzmaterials auf menschliche Knochenzellen nicht zu übersehen, die mit einem Zelltod einher gehen würden.

Primäre menschliche osteoblastäre Knochenzellen sind vor einem klinischen Einsatz neuer Knochenersatzmaterialien in vivo daher als sensibles Testsystem geeignet, um die Wirkung eines neuartigen Knochenersatzmaterials auf den humanen Knochenzellstoffwechselzu untersuchen.

Eine Zelle in vitro - so auch eine primäre menschliche Knochenzelle - hat vier mögliche Reaktionsweisen:
- keinerlei Reaktion
- Apoptose (Zelltod)
- vermehrte / verminderte Zellteilung
- vermehrte / verminderte Produktion von differenzierten Zellprodukten (zum Beispiel bei Knochenzellen alkalische Phosphatase), die für den Aufbau neuen Knochengewebes und für die Mineralisierung und Hydroxylapatitbildung in vivo notwendig ist..

Zum Durchführen der Versuche wurden Kokulturen von primären menschlichen Knochenzellen verwendet. Zum Vergleich der Wirkung wurden kommerziell verfügbare Knochenersatzmaterialien sowie osteotropes Knochenersatzmaterial, welches entsprechend dem erfindungsgemäßen Verfahren hergestellt wurde, verwendet. Bei den in vitro Experimenten wurden folgende Knochenersatzstoffe hinsichtlich ihrer Wirkung auf primäre menschliche Knochenzellen vergleichend ausgetestet:
1. BioOss^{®} (bovines Knochen-Granulat, kommerziell von Geistlich Biomaterials vertrieben)
2. Algipore^{®} (basierend auf EP 230 570 B1, kommerziell von Dentsply vertrieben)
3. Neues Algipore 1 (= NA1) erfindungsgemäßes osteotropes Knochenersatzmaterial
4. Neues Algipore 2 (= NA2) erfindungsgemäßes osteotropes Knochenersatzmaterial
5. Neues BioOss1 (BioOss^{®}, welches dem erfindungsgemäßen Verfahren unterzogen wurde)

| | NA1 | NA2 | BioOss1 |
|---|---|---|---|
| Ausgangsmaterial [g] | 7.21 | 7.51 | 0.5 |
| Dauer [h:min] | 116.9666667 | 221.75 | 116 |
| Ammoniumhydrogenphosphat [g] | 10 | 10.001 | 10.0521 |
| Kaliumfluorid [g] | 0.4 | 0.821 | 1,996 |
| SrF2 [g] | 0.35 | 0.688 | 0.821 |
| Ammoniaklösung 25% [g] | 6g | 50,278 | 18.183 |
| H2O | auf 75% aufgefüllt | auf 75% aufgefüllt | auf 75% aufgefüllt |
| Autoklavvolumen [ml] | 57.7 | 57.7 | 57.7 |

Es wurden folgende Bestimmungsmethoden zur Untersuchung des Einflusses der obigen Materialien auf Zellfunktionen von primären menschlichen Knochenzellen ausgewählt:
a. Alkalische Phosphatase
b. Zellprotein

### 1. Versuch

### Alkalische Phosphatase

Die vergleichenden Ergebnisse werden in % der Kontrolle +/- Standardabweichung ausgedrückt. Zunächst wurde die Aktivität der alkalischen Phosphatase im Medium-überstand nach Kultur der menschlichen Knochenzellen in Anwesenheit der verschiedenen Materialien untersucht. Als Kontrolle diente ein Aliquot des verwendeten Zellkultur-Nährmediums alleine ohne Zellen, da in den Löchern der Kulturplatten stets Reste des im Kulturmediums enthaltenen Serums trotz serumfreien Spülens vor der Exposition der Zellen zu den Materialien zurück bleiben. Das Serum enthält stets auch geringe Mengen alkalische Phosphatase. Bei diesem ersten Versuch stand das neue BioOss1 noch nicht zur Verfügung.

| Zellen alleine | BioOss^{®} | Algipore^{®} | NA1 | NA2 |
|---|---|---|---|---|
| 243+/-9 % | 216+/-3 % | 240+/-8 % | 291+/-9 % | 443+/-15 % |

Diese Ergebnisse sind in Fig. 1 graphisch verdeutlicht.

### Interpretation

BioOss^{®} vermindert eher die Aktivität des für die Mineral- und Knochenbildung menschlicher Knochenzellen unverzichtbaren Enzyms alkalische Phosphatase, während das erfindungsgemäße Knochenersatzmaterial eine hochsignifikant gesteigerte Aktivität der von menschlichen Knochenzellen sezernierten alkalischen Phosphatase bewirkt.

Die knochenspezifische alkalische Phosphatase als das wichtigste Osteoblasten-Marker Protein wird also durch das erfindungsgemäße Knochenersatzmaterial höchst signifikant stärker im menschlichen Knochenzellmodell stimuliert , als bei der Anwesenheit der herkömmlichen Materialien. Die unterschiedliche Wirkstärke von NA1 und NA2 auf die alkalische Phosphataseaktivität ist zurückführbar auf einen erhöhten Fluorid und Strontiumgehalt in NA2 gegenüber NA1.

Bei der Messung der Aktivität der alkalischen Phosphatase im Zellkulturüberstand ist zu berücksichtigen, dass auch in vivo die Mineral- bzw. Knochenbildung extrazellulär durch die Sekretion der alkalischen Phosphatase in das osteoblastäre Mikromilieu erfolgt.

### Zellteilung / Zellprotein

Als einen Hinweis auf eine Wirkung der Knochenersatzmaterialien auf die Zellteilung wurde der Proteingehalt in den einzelnen "Löchern", das heißt Reaktionskammern, der verwendeten Multilochplatten analysiert. Dazu wurde ein Tritonextrakt aus den jeweiligen Löchern für die Bestimmung des Proteingehaltes nach dem BCA-Verfahren verwendet. Je höher der Proteingehalt in den einzelnen Löchern, umso mehr Zellmaterial, welches Proteinmaterial entspricht, muss in den Löchern vorhanden sein. Dies bedeutet, dass sich die Zellzahl erhöht hat, da eine Knochenzelle stets eine ähnliche Größe aufweist und Proteine nicht in größerem Ausmaß intrazellulär in Knochenzellen gespeichert werden. Eine Abnahme des Proteingehaltes in einem Zellkulturloch, also einer Reaktionskammer, wäre also gleichbedeutend mit einer Abnahme der Zellzahl, die adhärent am Zellkulturboden oder auf den Knochenersatzstoffen sitzen (apoptotische, d.h. abgestorbene Zellen, bleiben nicht adhärent und werden vor der Triton-Zugabe weggespült). Die Ergebnisse werden wieder in % der Kontrolle +/- Standardabweichung angegeben.

| Zellen alleine | BioOss^{®} | Algipore^{®} | NA1 | NA2 |
|---|---|---|---|---|
| 96+/-3 % | 108+/-4 % | 103+/-3 % | 103+/-3 % | 103+/-3 % |

Diese Ergebnisse sind in Fig. 2 graphisch verdeutlicht.

### Interpretation

Es gibt keinen Unterschied zwischen den untersuchten Materialien auf den Zellproteingehalt in den Zellkulturlöchern. Daher beeinflussen die Materialien die Zellteilung nicht, aber auch den Zelltod nicht, da apoptotisch degenerierende Zellen nicht adhärent bleiben, sondern sich ablösen und vor dem Assay-Verfahren weggespült würden.

### Gesamtbetrachtung

Diese Beobachtungen deuten darauf hin, dass die nach erfindungsgemäßen Verfahren gewonnenen Materialien einen sehr positiven Effekt auf die alkalische Phosphatase Aktivität menschlicher Knochenzellen haben.

Diese Beobachtung ist vereinbar mit einem sehr günstigen und nachhaltigen, die Knochenmineralbildung in vivo stimulierenden Effekt des erfindungsgemäßen Knochenersatzmaterials, ohne dass die Zellteilung beeinflusst, also stimuliert oder gehemmt wird.

### 2. Versuch, Aktivierung des konventionellen BioOss^{®}-Materials durch das erfindungsgemäße Verfahren

Das bisher als Knochenersatzmaterial in der Zahnheilkunde oder Kieferchirurgie eingesetzte Material BioOss^{®} besteht aus dem natürlichen Hydroxylapatitmaterial des anorganischen Knochengewebes von Rindern. Mit dem erfindungsgemäßen Verfahren kann auch bei einem natürlichen Calciumphosphat Kristallgitter ein teilweiser Austausch von Calciumionen im Hydroxylapatitkristall mit Strontium- und Fluoridionen kontrolliert durchgeführt werden. Hierbei sollten Calcium- und Phosphor-Atome in der Gesamtmischung der Autoklaveneinwaage höchstens im Verhältnis 10:5 vorliegen.

In dem folgenden Experiment mit primären humanen osteoblastären Zellen wird die Wirkung des konventionellen BioOss^{®} Materials parallel zu den Wirkungen des durch das erfindungsgemäßen Verfahren aktivierten BioOss1 Materials auf die alkalische Phosphatase Aktivität untersucht.

Zugleich werden auch die oben bereits getesteten Materialien im gleichen Experiment eingesetzt, um eine Einordnung der Wirksamkeiten aller mit dem erfindungsgemäßen Verfahren hergestellten Materialien in einem parallelen Versuchsansatz zu ermöglichen. Es wurde auch hier die alkalische Phosphatase Aktivität im Zellkulturüberstand nach einer Inkubationszeit der Zellen mit dem neuen Material von 24 Stunden gemessen.

Dieses Experiment wurde wieder mit primären menschlichen Knochenzellen eines anderen Individuums als bei den ersten Experimenten (1. Versuch) durchgeführt.

### Alkalische Phosphatase

| Zellen alleine | BioOss^{®} | BioOss1 | Algipore^{®} | NA1 | NA2 |
|---|---|---|---|---|---|
| 393+/-19 % | 368+/-16 % | 645+/-20 % | 431 +/-24 % | 418+/-9 % | 747+/-51 % |

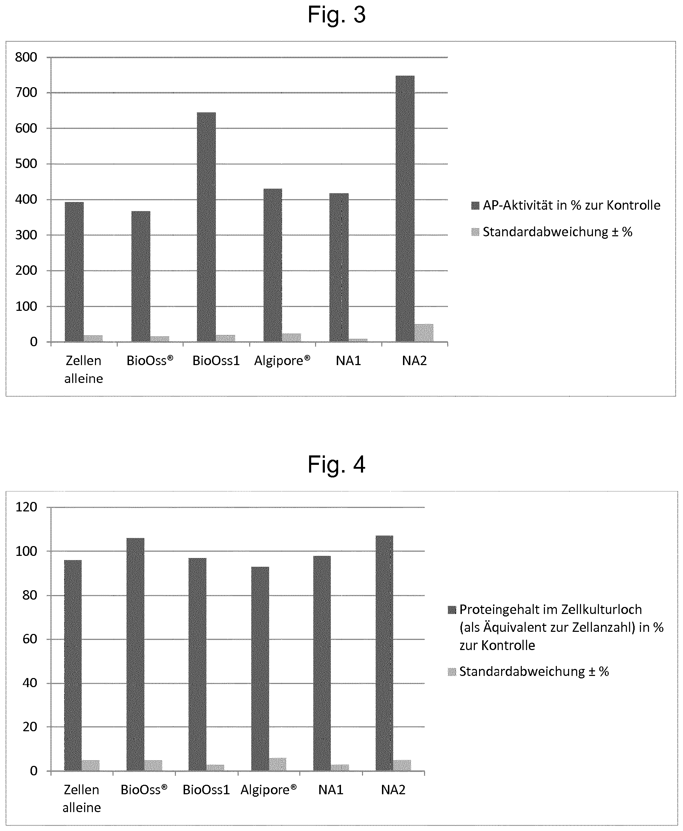

Diese Ergebnisse sind in Fig. 3 graphisch verdeutlicht.

### Zellteilung / Zellprotein

| Zellen alleine | BioOss^{®} | BioOss1 | Algipore^{®} | NA1 | NA2 |
|---|---|---|---|---|---|
| 96+/-5 % | 106+/-5 % | 97+/-3 % | 93+/-6 % | 98+/-3 % | 107+/-5 % |

Diese Ergebnisse sind in Fig. 4 graphisch verdeutlicht.

### Interpretation

Während das konventionelle BioOss^{®} Material keine signifikant stimulierende Wirkung auf die alkalische Phosphatase Aktivität in den Zellkulturüberständen im Vergleich zu humanen Knochenzellen ohne Kontakt zu einem Knochenersatzmaterial hat, bewirkt das durch das erfindungsgemäße Verfahren vorbehandelte BioOss^{®} Material (BioOss1) fast eine Verdopplung der alkalischen Phosphatase Aktivität. Daher eignet sich das erfindungsgemäße Verfahren auch zur Aktivierung von kommerziell verfügbarem bovinen Hydroxylapatit-Material und verleiht dem bisher nur osteokonduktiven BioOss^{®} Material osteoinduktive Eigenschaften.

Im Übrigen reproduziert dieses Experiment die Ergebnisse des oben bereits analysierten ersten Versuchs: Kommerzielles BioOss^{®} zeigt keine wesentliche Stimulation der Alkalische Phosphatase, während das mit dem erfindungsgemäßen Verfahren hergestellte BioOss1 eine fast doppelt so starke Aktivierung der Alkalische Phosphatase-Aktivität zeigt. Auch die mit dem erfindungsgemäßen Verfahren hergestellten "Algenhydroxylapatitmaterialen" NA1 und insbesondere NA2 zeigen eine noch potentere Stimulation der Alkalische Phosphatase-Aktivität.

In diesem vergleichenden Experiment ergibt sich erneut kein sicherer Hinweis auf eine signifikant unterschiedliche Wirkung der getesteten Knochenersatzmaterialien auf die zelluläre Gesamt-Proteinproduktion in den Zellüberständen in den Reaktionskammern. (siehe Fig 4)

### 3. Versuch, Reproduktion der Ergebnisse

In einem weiteren Ansatz werden alle Experimente nochmals mit anderen primären humanen osteoblastären Zellen eines dritten gesunden Spenders wiederholt und die Ergebnisse konsistenter Weise reproduziert. Es werden hier also nochmals die Wirkungen aller bisher nach dem erfindungsgemäßen Verfahren hergestellten Materialien parallel in einem weiteren Versuchsansatz reproduziert und mit den Wirkungen kommerziell verfügbarer Knochenersatzstoffe verglichen.

Die alkalische Phosphatase Aktivitäten wurden wieder in den Zellkulturüberständen in den Reaktionskammern gemessen.

### Alkalische Phosphatase

| Zellen alleine | BioOss^{®} | BioOss1 | Algipore^{®} | NA1 | NA2 |
|---|---|---|---|---|---|
| 566+/-32 % | 488+/-14 % | 708+/-18 % | 683+/-31 % | 683+/-61 % | 867+/-63 % |

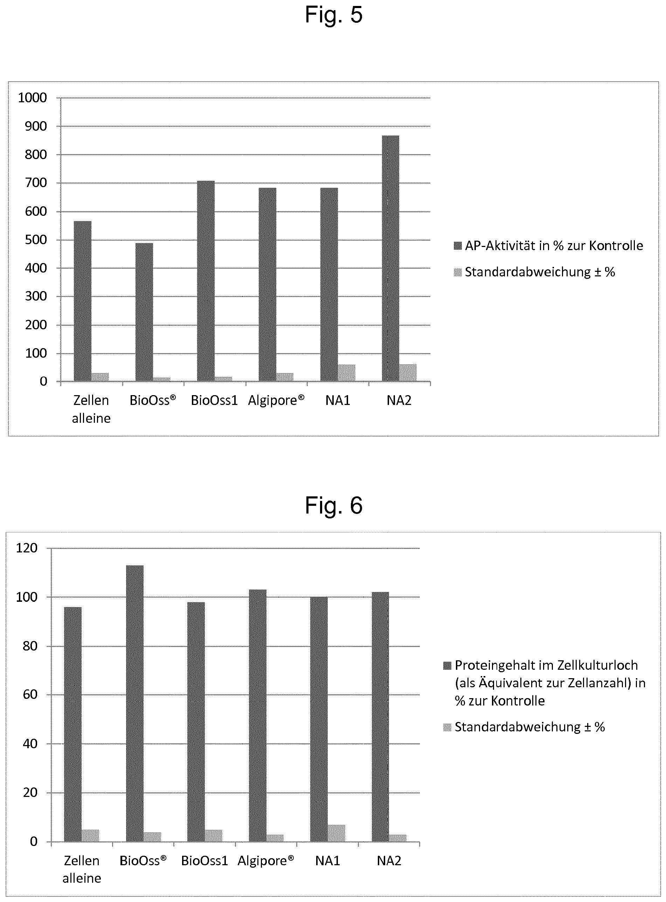

Diese Ergebnisse sind in Fig. 5 graphisch verdeutlicht.

### Zellteilung / Zellprotein

| Zellen alleine | BioOss^{®} | BioOss1 | Algipore^{®} | NA1 | NA2 |
|---|---|---|---|---|---|
| 96+/-5 % | 113+/-4 % | 98+/-5 % | 103+/-3 % | 100+/-7 % | 102+/-3 % |

Diese Ergebnisse sind in Fig. 6 graphisch verdeutlicht.

### Interpretation

Auch in diesem Experiment erweisen sich die durch das erfindungsgemäße Verfahren "aktivierten" Knochenersatzmaterialien NA2 und BioOss1 als signifikant stärker wirksam hinsichtlich der Stimulation der alkalischen Phosphatase Aktivität als die kommerziell verfügbaren Knochenersatzstoffe BioOss^{®} und Algipore^{®}. Das in einem initialen Prozess behandelte NA1 Material zeigt eine vergleichbare Wirkung wie das kommerziell erhältliche Algipore^{®} Knochenersatzmaterial.

Somit erweist sich jedes durch das erfindungsgemäße Herstellungsverfahren "aktivierte" Knochenersatzmaterial den bisherigen Produkten als hinsichtlich der Stimulation des osteoblastären Standard-Knochenanbaumarkers alkalische Phosphatase überlegen

Das erfindungsgemäße Herstellungsverfahren ist also geeignet, sowohl während eines Umsetzungsprozess aus einem Kalziumkarbonat beziehungsweise einer Mischung aus Portlandit, Ätzkalk, und Kalzit in ein Apatitmaterial, als auch direkt aus einem bestehendem Hydroxylapatitmaterial, ein aktiviertes Knochenersatzmaterial durch das Einfügen von aktivierenden Ionen, wie beispielsweise Strontiumionen, in das Kristallgitter herzustellen. Als aktiviert im Rahmen der Erfindung kann insbesondere ein Material angesehen werden, welches osteoinduktive beziehungsweise osteotrope oder auch antiresorptive Eigenschaften besitzt.

Somit wird mit dem erfindungsgemäßen Verfahren beziehungsweise mit dem durch das erfindungsgemäße Verfahren hergestellten osteotropen Knochenersatzmaterial ein Material geschaffen, welches langwirkende und örtlich begrenzte osteoinduktive beziehungsweise osteotrope Eigenschaften aufweist und sich hervorragend als Implantatmaterial im Knochengewebe eignet.

## Patentansprüche

1. Verfahren zum Herstellen eines osteotropen Knochenersatzmaterials aus einem
Ausgangsmaterial, welches im Wesentlichen Portlandit, Ätzkalk, Aragonit; Kalzit und/oder Apatit, insbesondere Hydroxylapatit, aufweist,
wobei Portlandit, Ätzkalk, Aragonit und/oder Kalzit in Form von gebrannten, ungebrannten und/oder chemisch aufbereiteten biogenen Skeletten verwendet werden oder
wobei als Apatit Wirbeltierknochen nach pyrolytischer oder chemischer Mazeration verwendet werden
wobei das Ausgangsmaterial mit einer Strontium-; Fluor- und/oder Galliumquelle in einen Autoklav eingebracht wird,
wobei beim Verwenden eines Ausgangsmaterials, welches im Wesentlichen Portlandit, Ätzkalk, Aragonit; Kalzit aufweist eine Phosphatquelle eingebracht wird,
wobei H₂O als Bestandteil eines Lösungsmittels in den Autoklav zugegeben wird,
wobei der pH-Wert in dem Autoklav in einem Bereich über 7 eingestellt wird,
wobei der geschlossene und gefüllte Autoklav für mindestens 1 Stunde erhitzt und anschließend abgekühlt wird, und
wobei anschließend der Inhalt des Autoklavs von Resten der Phosphor-, Strontium-, Fluor- und/oder Galliumquelle gereinigt wird.

2. Verfahren nach Anspruch 1,
**dadurch gekennzeichnet,**
**dass** der geschlossene und gefüllte Autoklav auf mindestens 30 Grad Celsius bevorzugt auf über 190 Grad Celsius erhitzt wird.

3. Verfahren nach Anspruch 1 oder 2,
**dadurch gekennzeichnet,**
**dass** der Inhalt des Autoklavs mechanisch mit einer Filtervorrichtung gereinigt wird.

4. Verfahren nach einem der Ansprüche 1 bis 3,
**dadurch gekennzeichnet,**
**dass** der Inhalt des Autoklavs mit H₂O bis zum Erreichen eines pH-Wertes von bevorzugt kleiner als 8 gewaschen wird.

5. Verfahren nach einem der Ansprüche 1 bis 4,
**dadurch gekennzeichnet,**
**dass** zum Einstellen des pH-Wertes in dem Autoklav in einem Bereich über 7 eine basische Lösung, insbesondere eine Ammoniak-Lösung, verwendet wird.

6. Verfahren nach einem der Ansprüche 1 bis 5,
**dadurch gekennzeichnet,**
**dass** die Strontium-, Fluor- und/oder Galliumquelle im Überschuss in Bezug auf das Ausgangsmaterial eingebracht wird.

7. Verfahren nach einem der Ansprüche 1 bis 6,
**dadurch gekennzeichnet,**
**dass** als Phosphorquelle Ammoniumdihydrogenphosphat oder Diammoniumphosphat zugegeben wird.

8. Verfahren nach Anspruch 7,
**dadurch gekennzeichnet,**
**dass** Calcium und Phosphoratome in der Gesamtmischung höchstens im Verhältnis 10:5 (Atomverhältnis) vorliegen.

9. Verfahren nach einem der Ansprüche 1 bis 8,
**dadurch gekennzeichnet,**
**dass** der geschlossene und gefüllte Autoklav für mindestens 12 Stunde erhitzt wird.

10. Verfahren nach einem der Ansprüche 1 bis 9,
**dadurch gekennzeichnet,**
**dass** als Strontium-, Fluor- und/oder Galliumquelle ein leicht auswaschbares oder schwer wasserlösliches Material verwendet wird.

11. Verfahren nach einem der Ansprüche 1 bis 10,
**dadurch gekennzeichnet,**
**dass** die Strontium-, Fluor- und/oder Galliumquelle als Feststoff in einem Behältnis in den Autoklav zugegeben wird, und
**dass** das Behältnis den Austausch von Ionen der Strontium-, Fluor- und/oder Galliumquelle mit dem Lösungsmittel ermöglicht, wobei Feststoffe zurückgehalten werden.

12. Verfahren nach einem der Ansprüche 1 bis 11,
**dadurch gekennzeichnet,**
**dass** das Ausgangsmaterial vor oder nach dem Einbringen in den Autoklav und/oder der Inhalt des Autoklavs einer pyrolytischen Behandlung und/oder einem chemischen Reinigungsverfahren unterzogen wird.

13. Osteotropes Knochenersatzmaterial, insbesondere nach einem Verfahren nach einem der Ansprüche 1 bis 12, welches im Wesentlichen Apatit aufweist,
• welches aus Portlandit, Ätzkalk, Aragonit und/oder Kalzit in Form von gebrannten, ungebrannten und/oder chemisch aufbereiteten biogenen Skeletten hergestellt ist oder
• welches aus Wirbeltierknochen nach pyrolytischer oder chemischer Mazeration hergestellt ist
wobei Strontium-, Fluor- und/oder Galliumionen in das Kristallgitter des Apatits eingebaut sind.

## Claims

1. Method for producing an osteotropic bone replacement material from a starting material which substantially has portlandite, calcium oxide, aragonite; calcite and/or apatite, in particular hydroxyl apatite,
wherein portlandite, calcium oxide, aragonite and/or calcite are used in the form of burnt, unburnt and/or chemically treated biogenic skeletons or
wherein as apatite vertebrate bones after pyrolytic or chemical maceration are used,
wherein the starting material is introduced into an autoclave with a strontium, fluorine and/or gallium source,
wherein when using a starting material which substantially has portlandite, calcium oxide, aragonite; calcite a phosphate source is introduced,
wherein H₂O is added into the autoclave as part of a solvent,
wherein the pH value in the autoclave is set to a range above 7,
wherein the closed and filled autoclave is heated for at least 1 hour and then cooled, and
wherein subsequently the content of the autoclave is cleaned from residues of the phosphorus, strontium, fluorine and/or gallium source.

2. Method according to claim 1,
**characterized in that**
the closed and filled autoclave is heated to at least 30 degrees Celsius, preferably to over 190 degrees Celsius.

3. Method according to claim 1 or 2,
**characterized in that**
the content of the autoclave is cleaned mechanically using a filter apparatus.

4. Method according to any one of claims 1 to 3,
**characterized in that**
the content of the autoclave is washed with H₂O until a pH value of preferably smaller than 8 is reached.

5. Method according to any one of claims 1 to 4,
**characterized in that**
to set the pH value in the autoclave to a range above 7 an alkaline solution, in particular an ammonia solution, is used.

6. Method according to any one of claims 1 to 5,
**characterized in that**
the strontium, fluorine and/or gallium source is introduced in excess in relation to the starting material.

7. Method according to any one of claims 1 to 6,
**characterized in that**
as phosphorus source ammonium dihydrogen phosphate or diammonium phosphate is added.

8. Method according to claim 7,
**characterized in that**
in the total mixture calcium and phosphorus atoms are present at a ratio not exceeding 10:5 (atomic ratio).

9. Method according to any one of claims 1 to 8,
**characterized in that**
the closed and filled autoclave is heated for at least 12 hours.

10. Method according to any one of claims 1 to 9,
**characterized in that**
as strontium, fluorine and/or gallium source a material easy to wash out or of bad water solubility is used.

11. Method according to any one of claims 1 to 10,
**characterized in that**
the strontium, fluorine and/or gallium source is added into the autoclave as solid matter in a container, and
**in that** the container enables the exchange of ions of the strontium, fluorine and/or gallium source with the solvent wherein solids are retained.

12. Method according to any one of claims 1 to 11,
**characterized in that**
prior to or after introduction into the autoclave the starting material and/or the content of the autoclave is subjected to a pyrolytic treatment and/or a chemical cleaning method.

13. Osteotropic bone replacement material, in particular pursuant to a method according to any one of claims 1 to 12, which substantially has apatite,
• which is produced from portlandite, calcium oxide, aragonite and/or calcite in the form of burnt, unburnt and/or chemically treated biogenic skeletons or
• which is produced from vertebrate bones after pyrolytic or chemical maceration,
wherein strontium, fluorine and/or gallium ions are incorporated into the crystal lattice of the apatite.

## Revendications

1. Procédé de fabrication d'une substance de substitution osseuse ostéotrope à partir d'une substance de départ qui contient essentiellement de la portlandite, de la chaux vive, de l'aragonite, de la calcite et/ou de l'apatite, en particulier de l'hydroxyapatite,
la portlandite, la chaux vive, l'aragonite et/ou la calcite étant utilisées sous la forme de squelettes biogènes calcinés, non calcinés et/ou traités chimiquement ou
en guise d'apatite étant utilisés des os de vertébrés après macération pyrolytique ou chimique,
selon lequel la substance de départ est introduite dans un autoclave avec une source de strontium, de fluor et/ou de gallium,
selon lequel, lors de l'utilisation d'une substance de départ qui contient essentiellement de la portlandite, de la chaux vive, de l'aragonite, est apportée une source de phosphate,
selon lequel de l'H₂O est ajoutée comme composant d'un solvant dans l'autoclave,
selon lequel la valeur pH dans l'autoclave est réglée sur une plage supérieure à 7,
selon lequel l'autoclave fermé et rempli est chauffé pendant au moins 1 heure, puis refroidi et
selon lequel le contenu de l'autoclave est ensuite nettoyé des restes de la source de phosphore, de strontium, de fluor et/ou de gallium.

2. Procédé selon la revendication 1
**caractérisé**
**en ce que** l'autoclave fermé et rempli est chauffé à au moins 30 degrés Celsius, de préférence à plus de 190 degrés Celsius.

3. Procédé selon la revendication 1 ou 2,
**caractérisé**
**en ce que** le contenu de l'autoclave est nettoyé mécaniquement avec un dispositif de filtrage.

4. Procédé selon une des revendications 1 à 3,
**caractérisé**
**en ce que** le contenu de l'autoclave est lavé avec de l'H₂O jusqu'à atteindre une valeur pH de préférence inférieure à 8.

5. Procédé selon une des revendications 1 à 4,
**caractérisé**
**en ce que**, pour régler la valeur pH dans l'autoclave sur une plage supérieure à 7, est utilisée une solution basique, en particulier une solution ammoniaquée.

6. Procédé selon une des revendications 1 à 5,
**caractérisé**
**en ce que** la source de strontium, de fluor et/ou de gallium est apportée en excès par rapport à la substance de départ.

7. Procédé selon une des revendications 1 à 6,
**caractérisé**
**en ce qu'**en guise de source de phosphore est ajouté du dihydrogénophosphate d'ammonium ou du phosphate de diammonium.

8. Procédé selon une des revendications 1 à 7,
**caractérisé**
**en ce que** les atomes de calcium et de phosphore sont présents dans le mélange dans un rapport de 10:5 maximum (rapport atomique).

9. Procédé selon une des revendications 1 à 8,
**caractérisé**
**en ce que** l'autoclave fermé et rempli est chauffé pendant au moins 12 heures.

10. Procédé selon une des revendications 1 à 9,
**caractérisé**
**en ce qu'**en guise de source de strontium, de fluor et/ou de gallium est utilisée une substance facilement lavable ou difficilement soluble dans l'eau.

11. Procédé selon une des revendications 1 à 10,
**caractérisé**
**en ce que** la source de strontium, de fluor et/ou de gallium est ajoutée sous forme solide dans un récipient dans l'autoclave et
que le récipient permet l'échange d'ions de la source de strontium, de fluor et/ou de gallium avec le solvant, les particules solides étant retenues.

12. Procédé selon une des revendications 1 à 11,
**caractérisé**
**en ce que** la substance de départ avant ou après l'introduction dans l'autoclave et/ou le contenu de l'autoclave sont soumis à un traitement pyrolytique et/ou à un procédé de nettoyage chimique.

13. Substance de substitution osseuse ostéotrope, en particulier selon un procédé selon une des revendications 1 à 12, qui contient essentiellement de l'apatite
• qui est fabriquée à partir de portlandite, de chaux vive, d'aragonite et/ou de calcite sous la forme de squelettes biogènes calcinés, non calcinés et/ou traités chimiquement ou
• qui fabriquée à partir d'os de vertébrés après macération pyrolytique ou chimique,
dans laquelle des ions de strontium, de fluor et/ou de gallium sont intégrées dans le réseau cristallin de l'apatite.
